# EUROPÄISCHE PATENTSCHRIFT

(11) **EP 3 396 091 B1**
(45) Veröffentlichungstag und Bekanntmachung des Hinweises auf die Patenterteilung: **17.08.2022**
(21) Anmeldenummer: 18000153.9
(22) Anmeldetag: 16.02.2018
(51) Int. Cl.: E05C 3/04, E05C 7/02, E05C 19/16, E05B 65/00, C12M 1/00

(54) **LABORSCHRANK**
LABORATORY CABINET
ARMOIRE DE LABORATOIRE

(30) Priorität: 28.04.2017 DE 102017109262
(43) Veröffentlichungstag der Anmeldung: 31.10.2018
(73) Patentinhaber: Binder GmbH, 78532 Tuttlingen (DE)
(72) Erfinder: Baumgärtner, Eugen, 78050 Villingen-Schwenningen (DE); Efinger, Patrick, 78582 Balgheim (DE); Storz, Ewald, 78604 Rietheim-Weilheim (DE); Binder, Peter Michael, 8595 Altnau (CH)
(74) Vertreter: Westphal, Mussgnug & Partner Patentanwälte mbB

(56) Entgegenhaltungen:
- WO-A1-2015/072793
- DE-C1- 19 803 601
- US-A- 4 771 269
- US-A1- 2013 026 900

## Beschreibung

Die Erfindung betrifft einen Laborschrank . Bekannt sind Laborschränke mit einem wenigstens eine Außentür aufweisenden Gehäuse, welches wenigstens einen Innenraum aufweist. Oft weisen derartige Laborschränke Vorrichtungen auf, mit welchen im Innenraum eine definierte Temperatur und/oder definierte Klimabedingungen, beispielsweise eine definierte Feuchte, einstellbar sind. Beispielsweise sind Kälteschränke, Wärmeschränke, Trockenschränke oder Brutschränke bekannt. Um zu verhindern, dass beim Öffnen der Außentür die klimatischen Bedingungen im Innenraum gestört werden, ist es bekannt, den Innenraum durch eine Innentür zu verschließen. Die Außentüre dient im Wesentlichen der Dämmung zwischen den Innenraum des Laborschranks und der Umgebung. Die Innentür verhindert den Luftaustausch des Innenraums mit der Umgebung beim Öffnen der Außentüre. Die Innentür kann aus transparentem Material gefertigt sein, was es ermöglicht, bei geöffneter Außentür einen Blick in den Innenraum zu werfen, ohne die klimatischen Bedingungen im Innenraum zu stören.

Bei den bekannten Laborschränken ist es erforderlich, dass ein Benutzer zunächst die Außentür des Laborschranks öffnet und anschließend mit einem zweiten Handgriff die Innentür öffnen kann.

US 2013 026 900 A1 beschreibt einen Kühlschrank mit einem wenigstens eine Außentür aufweisenden Gehäuse, welches wenigstens einen Innenraum aufweist, der durch eine Innentür verschließbar ist. Die Innentür des Kühlschranks umfasst eine Verriegelungsvorrichtung, welche mit Rasthaken, die an der Außentür angeordnet sind, zum Verriegeln der Außentür an der Innentür eingreift. Die Verriegelungsvorrichtung ist ein Druck-Zug-Knopf, bei dem die Rasthaken durch einen primären Pressvorgang in der Verriegelungsvorrichtung gefangen werden und der Verriegelungszustand der Rasthaken durch einen sekundären Pressvorgang gelöst wird. Dadurch ist es möglich, durch einen sekundären Pressvorgang die Außentür ohne die Innentür zu öffnen. Die Aufgabe der Erfindung besteht darin, einen Laborschrank dahingehend weiterzubilden, dass er benutzerfreundlicher zu handhaben ist und insbesondere das Öffnen der Innentür vereinfacht wird.

Die Aufgabe der Erfindung wird gelöst durch einen Laborschrank mit den Merkmalen des Patentanspruchs 1.

Vorteilhafte Ausgestaltungen und Weiterbildungen der Erfindung sind in den abhängigen Ansprüchen angegeben.

Der erfindungsgemäße Laborschrank, beispielsweise Kälteschrank, Wärmeschrank, Trockenschrank oder Brutschrank, mit einem wenigstens eine Außentür aufweisenden Gehäuse, welches wenigstens einen Innenraum aufweist, der durch eine Innentür verschließbar ist, zeichnet sich dadurch aus, dass der Laborschrank verstellbare Mittel aufweist und in Abhängigkeit von der Stellung der Mittel die Innentür gemeinsam mit der Außentür oder unabhängig von der Außentür öffenbar ist, wobei die Mittel bei geschlossener Außentür unzugänglich sind. Ein derartig ausgestalteter Laborschrank ermöglicht es dem Benutzer, gezielt einzustellen, ob beim Öffnen der Außentür die Innentür mit geöffnet werden soll oder nicht, verhindert aber ein unbefugtes oder unbeabsichtigtes Ändern dieser Einstellung.

Vorteilhafterweise sind die Mittel als verstellbarer Riegel ausgebildet, welche einfach zu bedienen und wenig störanfällig sind.

Besonders bevorzugt sind die Mittel als schwenkbarer Riegel ausgebildet, welcher platzsparend angeordnet sein kann.

Der Riegel kann beispielsweise einen Bügel hintergreifen, wobei der Bügel insbesondere sowohl beidseitig als auch nur einseitig befestigt und damit insbesondere einseitig offen ausgebildet sein kann. Der Bügel kann schwenkbar ausgebildet sein. Eine bevorzugte Weiterbildung der Erfindung sieht vor, dass die Innentür einen Innentürverschluss aufweist und die Mittel durch den Innentürverschluss gebildet sind. Der Innentürverschluss ist derart ausgebildet, dass er in einer geöffneten Position das Verschwenken der Innentür gegen das Gehäuse ermöglicht und in einer geschlossenen Position die Innentür gegen Verschwenken relativ zum Gehäuse sichert. Der Innentürverschluss ist somit verstellbar und ermöglicht in Abhängigkeit von der Stellung der Mittel ein Öffnen der Innentür gemeinsam mit der Außentür oder unabhängig von der Außentür. In dem Fall, dass der Innentürverschluss vor dem Schließen der Außentür geschlossen wird, kann die Außentür unabhängig von der Innentür geöffnet und geschlossen werden. In dem Fall, dass der Innentürverschluss in der geöffneten Position verbleibt, wird durch eine zusätzliche Vorrichtung in Form eines Mitnehmers ein gemeinsames Öffnen der Innentür mit der Außentür ermöglicht.

Erfindungsgemäß ist zwischen der Außentür und der Innentür ein lösbarer Mitnehmer ausgebildet. Ein Mitnehmer bewirkt dabei eine Kopplung zwischen Außentür und Innentür, die zur Folge hat, dass beim Öffnen der Außentür die Innentür mitgenommen, insbesondere mitgezogen, wird. Ist der Mitnehmer lösbar, kann wahlweise eingestellt werden, ob der Mitnehmer eine Kopplung zwischen der Außentür und der Innentür herstellt oder nicht.

Erfindungsgemäß ist der Mitnehmer als an einer von Außentür und Innentür angeordneter Permanentmagnet ausgebildet, welcher mit dem anderen von Außentür und Innentür oder mit einem an dem anderen von Außentür und Innentür angeordneten magnetischen Element zusammenwirkt. Durch einen derartig ausgestalteten Mitnehmer ergibt sich eine magnetische Kopplung zwischen Innentür und Außentür, welche vorteilhafterweise auch eine Relativbewegung zwischen Innentür und Außentür beim Schwenken der beiden Türen um unterschiedliche Drehpunkte ermöglicht, da die magnetische Kopplung derart ausgebildet sein kann, dass der Permanentmagnet über das magnetische Element gleitet, um die Relativbewegung zwischen den beiden Türen auszugleichen.

Eine bevorzugte Ausführungsform der Erfindung sieht vor, dass die Innentür zumindest abschnittsweise aus transparentem Material, insbesondere Glas, gefertigt ist. Dies ermöglicht eine Einsicht in den Innenraum, ohne dass die Innentür geöffnet wird und damit die klimatischen Bedingungen im Innenraum gestört werden.

Die Erfindung wird anhand der nachfolgenden Figuren ausführlich erläutert. Es zeigen
- Figur 1: eine perspektivische Ansicht eines ersten Ausführungsbeispiels eines Laborschranks mit geöffneter Außentür,
- Figur 2: eine perspektivische Ansicht des Laborschranks gemäß Figur 1 mit geöffneter Außentür und geöffneter Innentür,
- Figur 3: eine weitere perspektivische Ansicht des Laborschranks gemäß Figur 2,
- Figur 4: eine vergrößerte Darstellung des Innentürverschlusses des Laborschranks gemäß Figur 1 mit dem Innentürverschluss in einer geschlossenen Position,
- Figur 5: eine vergrößerte Darstellung des Innentürverschlusses des Laborschranks gemäß Figur 1 mit dem Innentürverschluss in einer geöffneten Position,
- Figur 6: eine perspektivische Ansicht des Innentürverschlusses gemäß Figur 4;
- Figur 7: eine Draufsicht auf den Innentürverschluss gemäß Figur 4,
- Figur 8: einen Längsschnitt entlang der Linie A-A durch den Innentürverschluss gemäß Figur 7
- Figur 9: eine perspektivische Ansicht eines zweiten Ausführungsbeispiels eines Laborschranks mit geöffneter Außentür,
- Figur 10: eine perspektivische Ansicht des Laborschranks gemäß Figur 9 mit geöffneter Außentür und geöffneter Innentür,
- Figur 11: eine weitere perspektivische Ansicht des Laborschranks gemäß Figur 10,
- Figur 12: eine vergrößerte Darstellung des Innentürverschlusses des Laborschranks gemäß Figur 9 mit dem Innentürverschluss in einer geschlossenen Position,
- Figur 13: eine vergrößerte Darstellung des Innentürverschlusses des Laborschranks gemäß Figur 9 mit dem Innentürverschluss in einer geöffneten Position,
- Figur 14: eine perspektivische Ansicht des Innentürverschlusses gemäß Figur 12,
- Figur 15: eine Draufsicht auf den Innentürverschluss gemäß Figur 12,
- Figur 16: einen Längsschnitt entlang der Linie A-A durch den Innentürverschluss gemäß Figur 15.

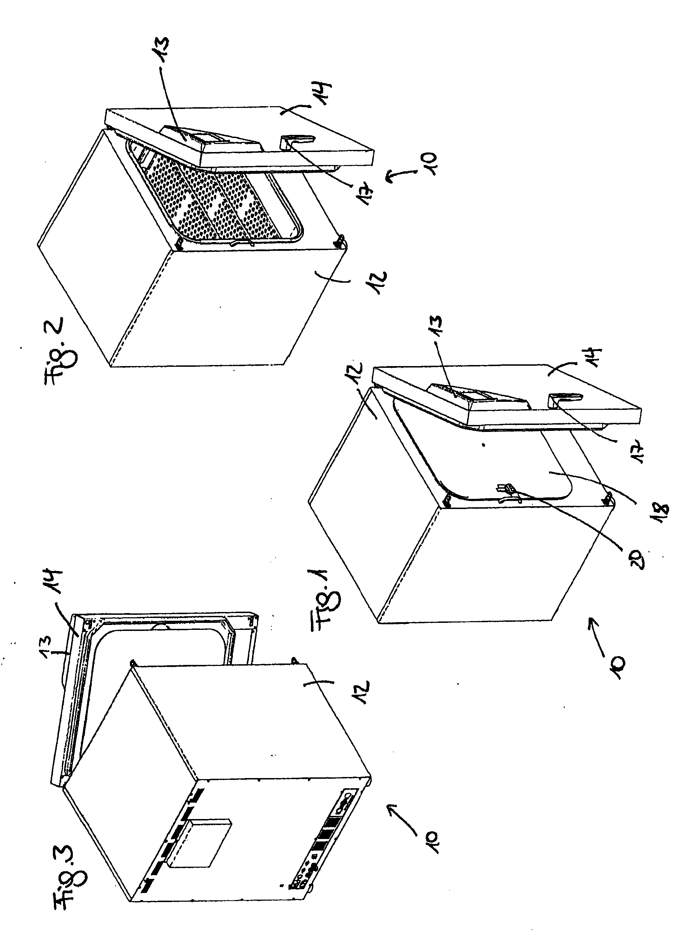

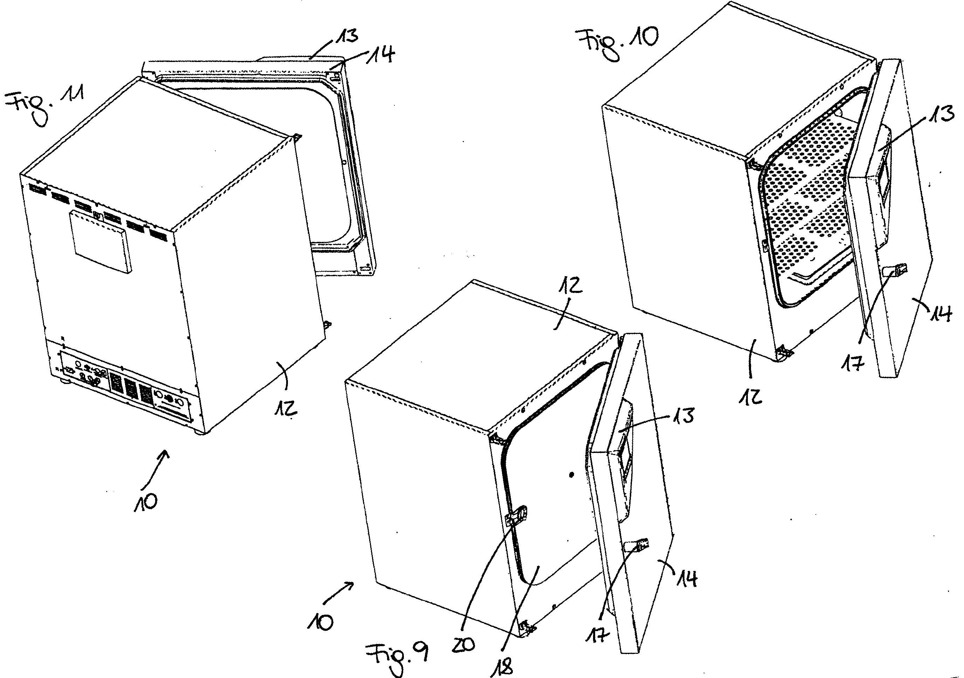

Die Figuren 1 bis 3 zeigen verschiedene perspektivische Ansichten eines ersten Ausführungsbeispiels Figuren 9 bis 11 zeigen verschiedene perspektivische Ansichten eines zweiten Ausführungsbeispiels eines Laborschranks 10, welcher beispielsweise als Kälteschrank, Wärmeschrank, Trockenschrank oder Brutschrank ausgebildet sein kann. Die nachstehenden Ausführungen gelten für beide Ausführungsbeispiele, soweit nicht ausdrücklich anders angegeben.

Der Laborschrank 10 weist ein Gehäuse 12 mit einer Außentür 14 auf, welches einen Innenraum umschließt. Das Gehäuse 12 kann eine Bodenseite, eine Deckseite, zwei gegenüberliegende Seitenwände und eine Rückwand aufweisen, wobei eine der Rückwand gegenüberliegende Öffnung durch die Außentür 14 verschlossen werden kann. Die Außentür 14 kann über einen Außentürverschluss geschlossen werden, welcher durch einen Handgriff 17 betätigt werden kann. Der Außentürverschluss sichert die Außentür 14 gegen zufälliges Verschwenken. Es kann ein zusätzlicher Sperrmechanismus vorgesehen sein, welcher das Entriegeln des Außentürverschlusses verhindert.

Das Gehäuse 12 kann zur besseren Wärmedämmung doppelwandig ausgebildet sein und insbesondere einen Innenkessel aufweisen. Der Laborschrank 10 weist wenigstens eine Innentür 18 auf, welche eine zur Außentür 14 weisende Öffnung des Innenraums verschließt, um einen Luftaustausch des Innenraums mit der Umgebung zu unterbinden oder zu verringern, wenn die Außentür 14 geöffnet wird.

Die Innentür 18 weist einen Innentürverschluss 20 auf, welcher die Innentür 18 in einer geschlossenen Position gegen Verschwenken sichert. Zusätzlich kann die Innentür 18 einen Sperrmechanismus aufweisen, welcher verhindert, dass der Innentürverschluss 20 geöffnet werden kann.

Die Innentür 18 kann zumindest abschnittsweise oder auch vollständig aus einem transparenten Material, beispielsweise Glas, gefertigt sein.

Es ist möglich, dass der Innenraum in mehrere Teilräume unterteilt ist, welche jeweils durch eine separate Innentür 18 verschlossen sind, so dass lediglich eine der Innentüren 18 geöffnet werden muss, wenn Probengut in den Laborschrank, insbesondere in einen der Teilräume, eingelagert oder aus dem Laborschrank 10 entnommen werden soll, dabei aber die klimatischen Bedingungen in den anderen Teilräumen möglichst wenig gestört werden.

An der Außenseite des Gehäuses 12 kann ein Bedienfeld 13 angeordnet sein, mit welchem insbesondere die klimatischen Bedingungen im Innenraum des Laborschranks 10, beispielsweise die Temperatur oder die Feuchte, eingestellt werden können.

Grundsätzlich ist es selbstverständlich möglich, dass der Laborschrank 10 auch zwei oder mehrere Außentüren 14 aufweisen kann.

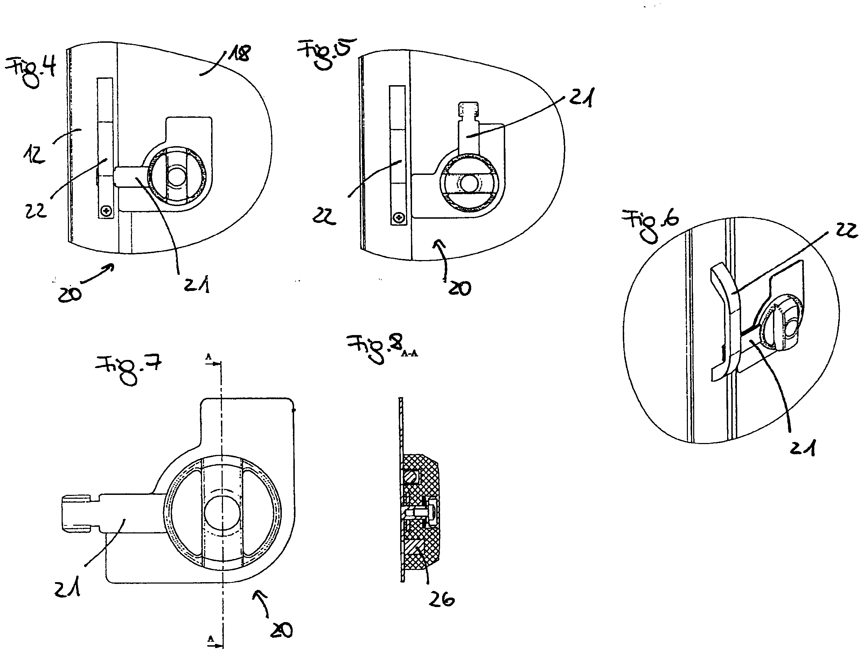

Die Figuren 4 bis 8 zeigen verschiedene Detailansichten des Innentürverschlusses 20 des in den Figuren 1 bis 3, die Figuren 12 bis 16 zeigen verschiedene Detailansichten des Innentürverschlusses 20 des in den Figuren 9 bis 11 dargestellten Laborschranks 10.

Der Laborschrank 10 weist verstellbare Mittel 20 auf, wobei in Abhängigkeit von der Stellung der Mittel 20 die Innentür 18 gemeinsam mit der Außentür 14 oder unabhängig von der Außentür 14 öffenbar ist, wobei die Mittel 20 bei geschlossener Außentür 14 unzugänglich sind.

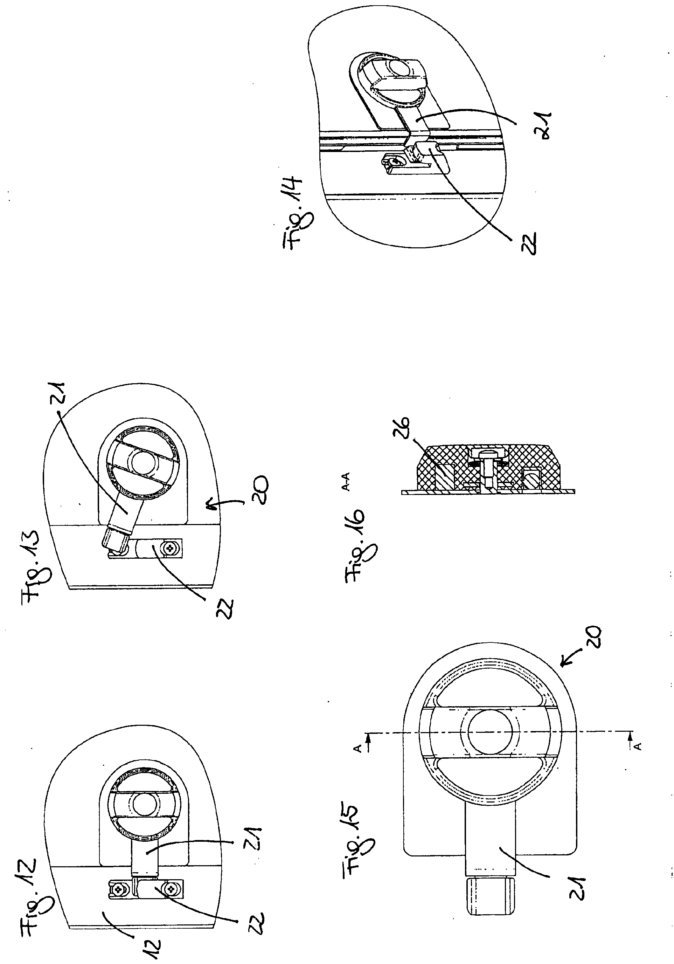

Die Mittel 20 können einen Riegel 21 aufweisen, welcher zwischen einer geöffneten Position (vgl. Figur 5 und Figur 13) und einer geschlossenen Position (vgl. Fig. 4 und Figur 12) verstellbar ist. Dazu greift der Riegel in der geschlossenen Position hinter einen Bügel 22 und ist in der geöffneten Position außer Eingriff mit dem Bügel 22. Der Bügel 22 kann dabei beidseitig befestigt (vgl. Figuren 4 bis 6) oder einseitig befestigt und einseitig offen (vgl. Figuren 12 bis 14) oder auch schwenkbar ausgebildet sein. Der Riegel 21 ist insbesondere an der Innentür 18 angeordnet, während der Bügel 22 an dem Gehäuse 12, insbesondere einer der Außentür 14 zugewandten Stirnseite einer der Seitenwände des Gehäuses 12, angeordnet ist. Durch diese Anordnung sind der Riegel 21 und der Bügel 22 durch die Außentür 14 abgedeckt, wenn die Außentür 14 geschlossen ist, so dass die Mittel 20 bei geschlossener Außentür 14 unzugänglich sind. Insbesondere ist der Riegel 21 als Drehriegel ausgebildet.

Es besteht die Möglichkeit, den Riegel 21 in der geöffneten Position durch einen Rastmechanismus zu sichern, um ein unbeabsichtigtes Verstellen des Riegels 21 zu erschweren.

Die Mittel 20 stellen vorzugsweise gleichzeitig einen Innentürverschluss dar, welcher die Innentür 18 in einer geschlossenen Position gegen Verschwenken relativ zum Gehäuse 12 sichert und in einer geöffneten Position das Verschwenken der Innentür 18 gegen das Gehäuse 12 erlaubt.

Zwischen der Außentür 14 und der Innentür 18 ist ein lösbarer Mitnehmer angeordnet, wobei der Mitnehmer als wenigstens ein Permanentmagnet 26 ausgebildet ist. Der Permanentmagnet 26 kann an der Innentür 18 oder der Außentür 14 angeordnet sein und ist im vorliegenden Ausführungsbeispiel an der Innentür 18, insbesondere an dem Riegel 21, angeordnet. Da der Innentürverschluss 20 bei geschlossener Außentür 14 an der Außentür 14 nahezu anliegt oder vorteilhafterweise auch in einer Mulde auf der Innenseite der Außentür 14 zu liegen kommen kann, und zudem die Innenseite der Außentür 14 aus einem magnetisierbaren Material gefertigt ist oder alternativ an der Innenseite der Außentür 14 ein magnetisierbares Element angeordnet ist, wird die Innentür 18 von der Außentür 14 magnetisch angezogen und bei Öffnen der Außentür 14 mitgenommen. Insbesondere kann die magnetische Kopplung zwischen der Innentür 18 und der Außentür 14 die Relativbewegung zwischen Innentür 18 und der Außentür 14 ausgleichen, indem der Permanentmagnet 41 über die Innenfläche der Außentür 14 gleitet.

Zur Bedienung der Türen 14, 18 bestehen unter anderem folgende Möglichkeiten: Ist vor dem Verschließen der Außentür 14 die Innentür 18 mittels der Mittel 20 verriegelt worden derart, dass der Riegel 21 in die geschlossene Position überführt wurde, kann ein Öffnen der Außentür 14 unabhängig von der Innentür 18 erfolgen, da beim Öffnen der Außentür 14 die magnetische Kopplung zwischen Innentür 18 und Außentür 14 gelöst wird, da die Innentür 18 durch den Riegel 21 in der geschlossenen Position gehalten wird. Ist vor dem Verschließen der Außentür 14 der Riegel 21 in die geöffnete Position überführt worden, wird beim Verschließen der Außentür 14 auch die Innentür 18 mit geschlossenen, beim Öffnen der Außentür 14 wird die Innentür 18 aber auch gleichzeitig aufgrund der magnetischen Kopplung zwischen der Innentür 18 und der Außentür 14 mitgenommen, so dass die Innentür 18 gemeinsam mit der Außentür 14 geöffnet wird.

### Bezugszeichenliste

- 10: Laborschrank
- 13: Bedienfeld
- 12: Gehäuse
- 14: Außentür
- 16: Außentürverschluss
- 17: Handgriff
- 18: Innentür
- 20: Mittel
- 21: Riegel
- 22: Bügel
- 26: Permanentmagnet

## Patentansprüche

1. Laborschrank (10), beispielsweise Kälteschrank, Wärmeschrank, Trockenschrank oder Brutschrank, mit einem wenigstens eine Außentür (14) aufweisenden Gehäuse (12), welches wenigstens einen Innenraum aufweist, der durch eine Innentür (18) verschließbar ist,wobei der Laborschrank (10)von dem Benutzer verstellbare Mittel (20) aufweist, die zwischen einer geöffneten Position und einen geschlossenen Position vor dem Verschließen der Außentür (14) verstellbar sind und in Abhängigkeit von der Stellung der Mittel (20) die Innentür (18) gemeinsam mit der Außentür (14) oder unabhängig von der Außentür (14) öffenbar ist, wobei die Mittel (20) bei geschlossener Außentür (14) unzugänglich sind, wobei zwischen der Außentür (14) und der Innentür (18) ein lösbarer Mitnehmer ausgebildet ist und wobei der Mitnehmer als an einer von Außentür (14) und Innentür (18) angeordneter Permanentmagnet (26) ausgebildet ist, welcher mit dem anderen von Außentür (14) und Innentür (18) oder mit einem an dem anderen von Außentür (14) und Innentür (18) angeordnetem magnetisierbaren Element zusammenwirkt.

2. Laborschrank nach Anspruch 1,
**dadurch gekennzeichnet, dass** die Mittel (20) als verstellbarer Riegel (21) ausgebildet sind.

3. Laborschrank nach einem der vorhergehenden Ansprüche,
**dadurch gekennzeichnet, dass** die Mittel als schwenkbarer Riegel (21) ausgebildet sind.

4. Laborschrank nach einem der vorhergehenden Ansprüche,
**dadurch gekennzeichnet, dass** die Innentür (18) einen Innentürverschluss aufweist und die Mittel (20) durch den Innentürverschluss gebildet sind.

5. Laborschrank nach einem der vorhergehenden Ansprüche,
**dadurch gekennzeichnet, dass** die Innentür (18) zumindest abschnittsweise aus transparentem Material, insbesondere Glas, gefertigt ist.

## Claims

1. Laboratory cabinet (10), for example a refrigeration cabinet, a heating cabinet, a drying cabinet or an incubator, with a housing (12), which comprises at least one outer door (14), and which comprises at least one inner chamber, which can be closed by means of an inner door (18), wherein the laboratory cabinet (10) comprises means (20), which can be adjusted by the user, which can be adjusted between an open position and a closed position before the closing of the outer door (14), and wherein, in dependency on the adjustment of the means (20), the inner door (18) can be opened together with the outer door (14) or independently of the outer door (14), wherein the means (20) are inaccessible when the outer door (14) is closed, wherein a releasable engagement is implemented between the outer door (14) and the inner door (18), and wherein the engagement is implemented as a permanent magnet (26), arranged on one of the outer door (14) and the inner door (18), which interacts with the other one of the outer door (14) and inner door (18), or with a magnetizable element arranged on the other one of the outer door (14) or the inner door (18).

2. Laboratory cabinet in accordance with claim 1,
**characterized in that** the means (20) are implemented as an adjustable bolt (21).

3. Laboratory cabinet in accordance with either of the preceding claims,
**characterized in that** the means are implemented as a swing bolt (21).

4. Laboratory cabinet in accordance with any of the preceding claims,
**characterized in that** the inner door (18) comprises an inner door lock and **in that** the means (20) are implemented by the inner door lock.

5. Laboratory cabinet in accordance with any of the preceding claims,
**characterized in that** the inner door (18) is, at least in part, made of a transparent material, in particular of glass.

## Revendications

1. Armoire de laboratoire (10), par exemple, armoire réfrigérante, armoire chauffante, armoire séchante ou incubateur, comprenant un caisson (12) avec au moins une porte extérieure (14), ayant un volume intérieur se fermant par une porte intérieure (18),
- l'armoire de laboratoire (10) ayant des moyens (20) réglables par l'utilisateur et qui sont réglables entre une position ouverte et une position fermée, avant de fermer la porte extérieure (14) et en fonction de la position des moyens (20), la porte intérieure (18) s'ouvre en commun avec la porte extérieure (14) ou indépendamment de la porte extérieure (14),
- les moyens (20) n'étant pas accessibles lorsque la porte extérieure (14) est fermée,
- un organe d'entraînement amovible étant réalisé entre la porte extérieure (14) et la porte intérieure (18),
- le moyen d'entraînement est sous la forme d'un aimant permanent (26) prévu sur la porte extérieure (14) ou la porte intérieure (18) et qui coopère avec l'autre porte, porte extérieure (14) ou porte intérieure (18) ou avec un élément magnétisable de l'autre porte extérieure (14) ou intérieure (18).

2. Armoire de laboratoire selon la revendication 1,
**caractérisée en ce que**
les moyens (20) sont sous la forme d'un verrou réglable (21).

3. Armoire de laboratoire selon l'une des revendications précédentes,
**caractérisée en ce que**
les moyens sont sous la forme d'un verrou pivotant (21).

4. Armoire de laboratoire selon l'une des revendications précédentes,
**caractérisée en ce que**
la porte intérieure (18) a une fermeture de porte intérieure et les moyens (20) sont formés par cette fermeture de porte intérieure.

5. Armoire de laboratoire selon l'une des revendications précédentes,
**caractérisée en ce que**
la porte intérieure (18) est au moins partiellement en une matière transparente, notamment en verre.
